# EUROPÄISCHE PATENTSCHRIFT

(11) **EP 0 415 192 B1**
(45) Veröffentlichungstag und Bekanntmachung des Hinweises auf die Patenterteilung: **02.11.1994**
(21) Anmeldenummer: 90115694.3
(22) Anmeldetag: 16.08.1990
(51) Int. Cl.: C07H 15/04

(54) **Verfahren zur Herstellung von Alkylglykosiden**
Process for the preparation of alkyl glycosides
Procédé de préparation d'alkylglycosides

(30) Priorität: 24.08.1989 DE 3927919
(43) Veröffentlichungstag der Anmeldung: 06.03.1991
(73) Patentinhaber: Henkel Kommanditgesellschaft auf Aktien, 40191 Düsseldorf (DE)
(72) Erfinder: Hill, Karlheiz, Dr., D-4006 Erkrath (DE); Weuthen, Manfred, Dr., D-4050 Mönchengladbach 1 (DE); Köhler, Hans-Peter, Dr., D-4000 Düsseldorf 13 (DE)

(56) Entgegenhaltungen:
- EP-A- 0 132 043
- DE-A- 1 905 523
- CHEMICAL ABSTRACTS, Band 91, 1979, Seite 612, Zusammenfassung nr. 157440v, Columbus, Ohio, US; & JP-A-79 84 537 (YOSHITOMO PHARMACEUTICAL INDUSTRIES, LTD) 05-07-1979

## Beschreibung

Die Erfindung betrifft ein neues, verbessertes Verfahren zur Herstellung von C₁-C₃₀-Alkylglykosiden unter Verwendung einer Säure als Katalysator.

Verfahren zur Herstellung von Alkylglykosiden unter Verwendung eines Katalysators sind bereits seit über 50 Jahren bekannt. So beschreibt die österreichische Patentschrift 135 333 die Herstellung von Laurylglucosid und Cetylglucosid aus Acetobromglucose und dem jeweiligen Fettalkohol in Gegenwart einer Base. Auch die Direktsynthese aus Glucose und Laurylalkohol mit Chlorwasserstoff als saurem Katalysator ist dort beschrieben.

In der US-amerikanischen Patentschrift 3 547 828 wird ein Herstellungsverfahren für oberflächenaktive Alkylglykoside beschrieben, in dem zunächst ein Saccharid mit einem niederen Alkohol wie Butanol in Anwesenheit eines sauren Katalysators zu einem Niedrigalkylglykosid umgesetzt wird, das anschließend mit einem höheren Alkohol zu dem höheren, oberflächenaktiven Alkylglykosid reagiert (Umacetalisierung). Geeignete saure Katalysatoren sind Mineralsäuren wie Schwefelsäure, Salzsäure und Salpetersäure, Paratoluolsulfonsäure und Methansulfonsäure.

Nach der Lehre der US-amerikanischen Patentschrift 3 598 865 werden C₈-C₂₅-Alkylglykoside durch die säurekatalysierte Umsetzung von Monosaccharid oder zu Monosaccharid hydrolysierbarem Oligo- oder Polysaccharid mit einem C₈-C₂₅-Alkohol in Gegenwart eines niederen Alkohols hergestellt. Der saure Katalysator ist Schwefelsäure, Salzsäure, Phosphorsäure, phosphorige Säure, Toluolsulfonsäure oder Bortrifluorid.

Die US-amerikanische Patentschrift 3 839 318 beschreibt ein Herstellungsverfahren von Alkylglykosiden durch direkte, säurekatalysierte Umsetzung eines höheren Alkohols mit einem Saccharid. Als geeignete Katalysatoren werden Mineralsäuren wie Schwefelsäure und Salzsäure und sulfosaure Ionenaustauscherharze genannt.

In der europäischen Patentanmeldung 132 043 wird bei der Herstellung von Alkylglykosiden, die durch Umsetzung einer Glykoseeinheit mit einem Alkohol erhalten werden (Direktsynthese), als saurer Katalysator die Säureform eines anionischen Tensids, wie Alkylhydrogensulfat, Alkylsulfonsäure und Alkylbenzolsulfonsäure, verwendet.

Die Erfindung geht von der Aufgabe aus, das Katalysatorpotential zur Herstellung von Alkylglykosiden zu erweitern.

Gegenstand der Erfindung ist ein Verfahren zur Herstellung von Alkylglykosiden der allgemeinen Formel RO(G)ₙ, wobei G eine Glykoseeinheit, n eine Zahl zwischen 1 und 10 und R einen aliphatischen Rest mit 1 - 30 Kohlenstoffatomen darstellt, unter Verwendung von Sulfobernsteinsäure als Katalysator.

Die erfindungsgemäß mit Sulfobernsteinsäure als Katalysator herstellbaren Alkylglykoside lassen sich durch die allgemeine Formel RO(G)ₙ beschreiben. Dabei leitet sich der aliphatische Rest R von einem Alkohol mit 1 - 30 Kohlenstoffatomen ab. Die höheren aliphatischen Alkohole werden vorzugsweise durch Reduktion natürlicher Fette gewonnen, so daß im Rahmen dieser Offenbarung der Begriff Alkyl in Alkylglykosid gesättigte und ethylenisch ungesättigte Reste und deren Gemische einschließlich solcher mit verschiedenen Kettenlängen im Gemisch umfaßt. Bevorzugte aliphatische Reste sind Alkylreste von linearen und primären Alkoholen mit 8 - 22 Kohlenstoffatomen und insbesondere von solchen mit 12 - 18 Kohlenstoffatomen. Ebenso eignet sich die Sulfobernsteinsäure zur Herstellung von Alkylglykosiden, deren Alkylrest sich von synthetischen primären Alkoholen ableitet, insbesondere von den sogenannten Oxoalkoholen, die nach der Methode der Oxosynthese bzw. Hydroformylierung erhalten werden und einen gewissen Prozentsatz, meist 20 - 40 %, an verzweigten Isomeren mit einem 2-Methylrest aufweisen. Typische Alkylglykoside, die mit dem erfindungsgemäßen Verfahren hergestellt werden können, sind solche, in denen Alkyl für Octyl, Nonyl, Decyl, Undecyl, Dodecyl, 2-Methylundecyl, Tridecyl, Tetradecyl, Pentadecyl, Hexadecyl, Heptadecyl, Octadecyl und Mischungen daraus besteht.

Die Glykoseeinheit G stammt von den üblichen Aldosen bzw. Ketosen, wie z. B. Glucose, Fructose, Mannose, Galactose, Arabinose und Ribose, oder von Oligo- und Polysacchariden ab, die sich durch Hydrolyse zu Monosacchariden abbauen lassen. Unter den Monosacchariden werden die Aldosen und insbesondere die Glucose wegen ihrer leichten Zugänglichkeit und Verfügbarkeit in technischen Mengen bevorzugt eingesetzt. Handelsübliche Glucose enthält häufig 1 Mol Kristallwasser. Bei Verwendung von Sulfobernsteinsäure als Katalysator zur Alkylglykosidherstellung können wasserfreie und kristallwasserhaltige Glucose gleichermaßen verwendet werden. Das wichtigste, zu einer Glucoseeinheit abbaubare Polysaccharid ist die Stärke. Pulverförmige Stärken und ihre partiellen Abbauprodukte, beispielsweise in Form eines entsprechenden, meist hochkonzentrierten Glucosesirups, sind ebenfalls als Ausgangsstoffe bevorzugt.

Die Indexzahl n ist eine beliebige Zahl zwischen 1 und 10; sie gibt den Oligomerisierungsgrad, d. h. die Verteilung von Monoglykosiden und Oligoglykosiden an. Während n in einer gegebenen Verbindung immer eine ganze Zahl sein muß und hier vor allem die Werte n = 1 - 6 annehmen kann, ist der Wert n für ein spezielles Produkt eine analytisch ermittelte rechnerische Größe, die meistens eine gebrochene Zahl darstellt.

Sulfobernsteinsäure kann in allen, insbesondere in allen technisch interessanten Verfahren zur säurekatalysierten Herstellung von Alkylglykosiden als Katalysator eingesetzt werden. In Frage kommen solche Verfahren, in denen Alkylglykoside entweder durch direkte Umsetzung eines Monosaccharids, insbesondere Glucose, mit einem Überschuß an aliphatischem Alkohol erhalten werden (Direktsynthese) oder in denen Alkylglykoside dadurch erhalten werden, daß ein Monosaccharid, insbesondere Glucose, oder ein zu einem Monosaccharid hydrolysierbares Oligo- oder Polysaccharid, insbesondere Stärke oder partielle Stärkeabbauprodukte, mit einem niederen, vorzugsweise 1 - 4 Kohlenstoffatome enthaltenden Alkohol oder Glykol zu einem Niedrigalkylglykosid umgesetzt wird, das anschließend mit einem höheren, vorzugsweise 8 - 22 Kohlenstoffatome enthaltenden aliphatischen Alkohol zu einem höheren, oberflächenaktiven Alkylglykosid reagiert (Umacetalisierung).

Durch den Einsatz von Sulfobernsteinsäure als Katalysator wird die Geschwindigkeit der Reaktion zwischen Mono-, Oligo- oder Polysaccharid einerseits und Alkohol, insbesondere aliphatischem Alkohol, andererseits (Direktsynthese sowie 1. Schritt des Umacetalisierungsverfahrens) ebenso wie die Reaktion des Niedrigalkylglykosids mit dem höheren aliphatischen Alkohol (2. Schritt des Umacetalisierungsverfahrens) in größerem Maße erhöht als durch den Einsatz von gleichen molaren Mengen herkömmlicher, bekannter Katalysatoren wie Mineralsäuren, beispielsweise Schwefelsäure, oder Paratoluolsulfonsäure. Die Reaktionszeit kann durch den Einsatz von Sulfobernsteinsäure - verglichen mit gleichen molaren Mengen herkömmlicher, bekannter Katalysatoren - um 40 bis 60 % verkürzt werden.

Ein weiterer, wesentlicher Vorteil einer Verwendung von Sulfobernsteinsäure als Katalysator in der Alkylglykosidherstellung besteht darin, daß bei gleichbleibender Reaktionszeit und/oder vorgegebener Umsatzmenge die eingesetzte molare Menge an Katalysator, bezogen auf 1 Mol Glykoseeinheit, gegenüber herkömmlichen Katalysatoren wie beispielsweise Schwefelsäure und Paratoluolsulfonsäure um mindestens 40 %, vorzugsweise bis 60 % und insbesondere bis 80 % gesenkt werden kann. Vorzugsweise werden sowohl in der Direktsynthese als auch im Umacetalisierungsverfahren und insbesondere im Umacetalisierungsverfahren ausgehend von Stärke bzw. partiellen Stärkeabbauprodukten Mengen von 1 bis 6 mMol Sulfobernsteinsäure, bezogen auf 1 Mol Glykoseeinheit, eingesetzt. Besonders bevorzugt sind Mengen von 1,5 bis 5 mMol und insbesondere 2,5 bis 3,5 mMol Sulfobernsteinsäure, bezogen auf 1 Mol Glykoseeinheit.

Der Einsatz von Sulfobernsteinsäure kann wasserfrei oder in Form einer wäßrigen Lösung, insbesondere in Form einer 60 - 80 Gew.-%igen klaren wäßrigen Lösung erfolgen.

Weitere, wesentliche Vorteile von Sulfobernsteinsäure bestehen in ihren ökologischen Eigenschaften. Sulfobernsteinsäure ist nicht aromatisch und biologisch leicht abbaubar.
Die erfindungsgemäß mit Sulfobernsteinsäure als Katalysator hergestellten Alkylglykoside besitzen den Vorteil, daß sie eine hellere Farbe und eine geringere Trübung als mit herkömmlichen Katalysatoren hergestellte Alkylglykoside aufweisen. Ebenso ist es von Vorteil, daß bei dem Einsatz geringerer Katalysatormengen in der üblicherweise in allen technisch interessanten Herstellungsverfahren vorhandenen Neutralisationsstufe entsprechend weniger Neutralisationsmittel aufgewendet werden muß. Die Reaktionsendprodukte sind dadurch mit einem geringeren Salzgehalt belastet als die Produkte, die mit herkömmlichen, üblichen Katalysatoren erhalten werden.

### Beispiele

### Beispiel 1

Dieses Beispiel beschreibt die Herstellung eines C₁₂-C₁₄-Alkylglucosids aus wasserfreier Glucose und einem technischen Fettalkohol (Gemisch aus circa 75 Gew.-% Dodecanol und circa 25 Gew.-% Tetradecanol) im Molverhältnis 1 : 4,5 nach der Direktsynthese.

Zu einer auf 115 °C aufgeheizten Mischung aus 838 g (4,32 Mol) eines Dodecanol-Tetradecanol-Gemisches (Lorol S^{(R)}, Henkel KGaA) und 180 g (1 Mol) wasserfreier Glucose (Puridex^{(R)}, Fa. Cerestar Deutschland GmbH) wurden 5.6 mMol Sulfobernsteinsäure, entsprechend 1,6 g einer 70 %igen Lösung in Wasser, die in 35 g (0,18 Mol) Lorol S gelöst waren, im Vakuum (20 mm Hg-Säule) zugegeben. Das mit fortschreitender Reaktion entstandene Wasser wurde im Vakuum abgetrennt, mittels Kühlung durch flüssigen Stickstoff kondensiert und in Abständen von 15 Minuten mengenmäßig bestimmt. Diese Werte wurden um 0,5 ml Wasser, die durch die wäßrige Sulfobernsteinsäurelösung eingeschleppt wurden, korrigiert. Daraus wurde der Reaktionsumsatz in Abhängigkeit von der Zeit berechnet (18 ml entsprachen einem Umsatz von 100 %). Die ermittelten Werte werden in Tabelle 1 wiedergegeben.

Zum Vergleich wurde der Versuch mit 5,6 mMol Paratoluolsulfonsäure-Monohydrat (1,1 g) als Katalysator wiederholt. Der ermittelte Reaktionsumsatz in Abhängigkeit von der Zeit wird ebenfalls in Tabelle 1 wiedergegeben.

**Tabelle 1**

| Katalysator | Umsatz nach Minuten | | | | | | |
|---|---|---|---|---|---|---|---|
| | 15 | 30 | 45 | 60 | 75 | 90 | 120 |
| Sulfobernsteinsäure | 55 % | 80 % | 94 % | 100 % | 100 % | 100 % | 100 % |
| Paratoluolsulfonsäure | 16 % | 58 % | 77 % | 91 % | 97 % | 99 % | 100 % |

### Beispiel 2

Dieses Beispiel beschreibt die Herstellung eines C₁₂-C₁₄-Alkylglucosids nach der Direktsynthese unter Verwendung von Glucose-Monohydrat.

Beispiel 1 wurde mit 198 g (1 Mol) Glucose-Monohydrat, das in Lorol S suspendiert 30 Minuten bei 80 °C und 15 mm Hg-Säule entwässert wurde, wiederholt. Die ermittelten Umsatzwerte entsprachen denen des Beispiels 1.

### Beispiel 3

Beispiel 1 wurde mit 2,8 mMol Sulfobernsteinsäure als Katalysator wiederholt. Die Reaktion war - wie im Vergleichsversuch mit 5,6 mMol Paratoluolsulfonsäure (siehe Tabelle 1) - nach 2 Stunden beendet.

### Beispiel 4

Dieses Beispiel beschreibt die Herstellung eines C₁₂-C₁₄-Alkylglucosids nach der Methode der Umacetalisierung ausgehend von Stärke und einem technischen Fettalkohol im Molverhältnis 1 : 4,5. 190,6 g (1 Mol) Kartoffelstärke (Wassergehalt 15 %) wurden mit 593 g (8 Mol) Butanol, 79,4 g (4,4 Mol) Wasser und 0,8 g (2,8 mMol) 70 %iger wäßriger Sulfobernsteinsäure unter Rühren und einem Druck von 6 bar auf 140 °C erhitzt. Nach 30 Minuten wurde die Reaktionsmischung auf 100 °C abgekühlt und das Wasser bei Normaldruck abdestilliert. Anschließend wurden 873 g (4,5 Mol) Lorol S (C₁₂-C₁₄-Fettalkohol) zugegeben und die Reaktionsmischung auf 110 °C aufgeheizt. Sei dieser Temperatur destillierte Butanol im Vakuum (20 mm Hg-Säule) ab. Nach 2 Stunden war die Butanoldestillation beendet.

Zum Vergleich wurde der Versuch mit Paratoluolsulfonsäure-Monohydrat als Katalysator wiederholt. Um die gleiche Reaktionszeit (2 Stunden) einhalten zu können, wurden 3,2 g (16,4 mMol) Paratoluolsulfonsäure-Monohydrat benötigt. Das Produkt war nach der Abdestillation von Butanol deutlich dunkler gefärbt als das mit Sulfobernsteinsäure erhaltene Produkt.

### Beispiel 5:

Beispiel 3 wurde mit 593 g (8 mol) Butanol wiederholt.

### Beispiel 6:

Beispiel 3 wurde mit 833,5 (4,5 mol) eines Dodecanol-Tridecanol-Gemisches (Neodol 23^{(R)}, Fa. Shell) wiederholt.

### Beispiel 7:

Beispiel 1 wurde mit 1091 g (4,5 mol) Hexadecanol (Lorol C₁₆^{(R)}, Henkel KGaA) und 4,2 mmol Katalysator (Sulfobernsteinsäure bzw. Paratoluolsulfonsäure-Monohydrat) wiederholt.

Die ermittelten Umsatzwerte in den Beispielen 5 bis 7 entsprachen denen der Beispiele 1 bis 3. In allen Versuchen war die Wirkung der Sulfobernsteinsäure als Katalysator signifikant stärker als die der Paratoluolsulfonsäure.

## Patentansprüche

1. Verfahren zur Herstellung von Alkylglykosiden der allgemeinen Formel RO(G)ₙ, wobei G eine Glykoseeinheit, n eine Zahl zwischen 1 und 10 und R einen aliphatischen Rest mit 1 - 30 Kohlenstoffatomen darstellt, nach der Methode der Direktsynthese oder der Umacetalisierung unter Verwendung von Sulfobernsteinsäure als Katalysator.

2. Verfahren nach Anspruch 1, dadurch gekennzeichnet, daß die Alkylglykoside durch Umsetzung eines Saccharids oder eines Niedrigalkylglykosids mit einem gesättigten linearen und primären Alkohol mit 8 - 22 Kohlenstoffatomen, vorzugsweise mit solchen mit 12 - 18 Kohlenstoffatomen, hergestellt werden.

3. Verfahren nach Anspruch 1 oder 2, dadurch gekennzeichnet, daß 1,5 bis 5 mMol, vorzugsweise 2,5 bis 3,5 mMol Sulfobernsteinsäure, bezogen auf ein Mol Glykoseeinheit, verwendet werden.

4. Verfahren nach einem der Ansprüche 1 - 3, dadurch gekennzeichnet, daß Sulfobernsteinsäure in Form einer 60 - 80 Gew.-%igen klaren wäßrigen Lösung eingesetzt wird.

## Claims

1. A process for the production of alkyl glycosides corresponding to the general formula RO(G)n, where G is a glycose unit, n is a number of 1 to 10 an R is an aliphatic C₁₋₃₀ radical, by the direct synthesis method or by the transacetalization method using sulfosuccinic acid as catalyst.

2. A process as claimed in claim 1, characterized in that the alkyl glycosides are prepared by reaction of a saccharide or a lower alkyl glycoside with a saturated linear and primary C₈₋₂₂ and preferably C₁₂₋₁₈ alcohol.

3. A process as claimed in claim 1 or 2, characterized in that 1.5 to 5 mmol and preferably 2.5 to 3.5 mmol sulfosuccinic acid is used per mol glycose unit.

4. A process as claimed in any of claims 1 to 3, characterized in that sulfosuccinic acid is used in the form of a 60 to 80% by weight clear aqueous solution.

## Revendications

1. Procédé de préparation d'alkylglycosides de la formule générale RO(G)ₙ, dans laquelle G représente une unité d'ose, n correspond à un nombre compris entre 1 et 10 et R est un radical aliphatique comportant 1 à 30 atomes de carbone, selon la méthode de la synthèse directe ou de la transacétalisation, moyennant l'utilisation d'acide sulfosuccinique comme catalyseur.

2. Procédé selon la revendication 1, caractérisé en ce que les alkylglycosides sont préparés par mise en réaction d'un saccharide ou d'un alkylglycoside inférieur avec un alcool linéaire et primaire saturé comportant 8 à 22 atomes de carbone, de préférence avec ceux comprenant 12 à 18 atomes de carbone.

3. Procédé selon la revendication 1 ou 2, caractérisé en ce que l'on utilise 1,5 à 5 mmoles, de préférence 2,5 à 3,5 mmoles, d'acide sulfosuccinique pour 1 mole d'unité d'ose.

4. Procédé selon une des revendications 1 à 3, caractérisé en ce que l'acide sulfosuccinique est mis en oeuvre sous la forme d'une solution aqueuse limpide de 60 à 80 % en poids.
